# EUROPEAN PATENT APPLICATION

(11) **EP 4 043 554 A1**
(43) Date of publication of application: **17.08.2022**
(21) Application number: 20875184.2
(22) Date of filing: 07.10.2020
(51) Int. Cl.: C12N 5/074

(54) **PRODUCTION METHOD FOR VASCULAR ENDOTHELIAL STEM CELL**

(30) Priority: 09.10.2019 JP 2019186035
(71) Applicant: OSAKA UNIVERSITY, Suita-shi Osaka 565-0871 (JP)
(72) Inventor: TAKAKURA, Nobuyuki, Suita-shi, Osaka 565-0871 (JP); NAITO, Hisamichi, Suita-shi, Osaka 565-0871 (JP); WAKABAYASHI, Taku, Suita-shi, Osaka 565-0871 (JP); IBA, Tomohiro, Suita-shi, Osaka 565-0871 (JP)
(74) Representative: Witte, Weller & Partner Patentanwälte mbB
(86) International application number: PCT/JP2020/038029
(87) International publication number: WO 2021/070874

(57) **Abstract**

The present invention provides a method for artificially producing a vascular endothelial stem cell from a non-vascular endothelial stem cell. The method for producing a vascular endothelial stem cell of the present invention comprises the step of bringing a vascular endothelial cell possessing no stem cell properties into contact with a factor secreted by an organ of a neonatal or juvenile mammal. The step may be a step of (1) transplanting the vascular endothelial cell possessing no stem cell properties into the organ of the neonatal or juvenile mammal, wherein the mammal is a non-human mammal, or (2) culturing the vascular endothelial cell possessing no stem cell properties in a culture system containing the factor secreted by the organ of the neonatal or juvenile mammal.

## Description

### TECHNICAL FIELD

The present invention relates to a method for producing a vascular endothelial stem cell.

### BACKGROUND ART

Vessel-related diseases are collectively referred to as vascular diseases, including, for example, malignant tumor, retinopathy, chronic inflammatory disease, arteriosclerosis, ischemic disease, and many others. In order to improve such diseases, angiogenesis control therapy is being actively developed. In particular, vascular regeneration therapy is called "therapeutic angiogenesis", which is a promising therapeutic strategy for diseases characterized by a decreased number of blood vessels or impaired vascular function resulting from various causes to regenerate blood vessels and improve pathological conditions. More specific examples are as follows: 1) a therapy to improve ischemia, such as myocardial ischemia, cerebral ischemia, and lower limb ischemia, by inducing new blood vessel formation, that is, a therapy to improve blood supply, which is an original function of blood vessels; 2) a vascular regeneration therapy for diseases characterized in that secretion of factors normally secreted by vascular endothelial cells is reduced by genetic or acquired causes (e.g., hemophilia, which develops as a result of reduced production of coagulation factors from vascular endothelial cells) ; and 3) vascular regeneration to normalize blood vessels in diseased organs and restore organ function (e.g., vascular regeneration for liver fibrosis, cirrhosis, atelectasis, osteoporosis, renal dysfunction, etc.).

The efficacy of therapeutic angiogenesis for the aforementioned diseases is beginning to be proven in basic medicine. In clinical practice, this strategy has actually been applied to cell therapy and cytokine (gene) therapy. For example, in cell therapy, one approach is to use vascular endothelial progenitor cells present in bone marrow to regenerate blood vessels. However, blood vessels formed from these progenitor cells are unlikely to be maintained for a long time because these progenitor cells are not stem cells. Another approach is to inject mesenchymal stem cells from adipose tissue or other sources to induce vascular regeneration. In this approach, angiogenesis-inducing substances secreted by mesenchymal stem cells are considered to induce proliferation of vascular endothelial cells in preexisting vessels and thus regeneration of blood vessels. However, this approach is not available for patients with a reduced ability to regenerate blood vessels.

Besides cell therapy, administration of a growth factor for proliferating vascular endothelial cells was also implemented. Vascular endothelial growth factor (VEGF) is a cytokine capable of inducing strongest proliferation of vascular endothelial cells. Around the year 2000, injection of a plasmid expressing a gene encoding VEGF into an ischemic region was implemented as vascular regeneration therapy. However, VEGF increased vascular permeability, causing edema due to massive accumulation of blood components in tissues. Because of this side effect, VEGF-based therapy is no longer used. On the other hand, hepatocyte growth factor (HGF) gene is clinically used for vascular regeneration therapy. There are some prospective data showing its efficacy, but no reference has been made to its long-term effects.

As with the case where hematopoietic stem cells are required to reconstitute bone marrow using another person's bone marrow by means of bone marrow transplantation, cells possessing stem cell properties are required to regenerate blood vessels with the aim of maintaining the regenerated vessels for a long period of time. The present inventors found that vascular endothelial cells in preexisting blood vessels include a very small percentage of cells that are highly capable of generating vascular endothelial cells (Non-Patent Literature 1 and 2). These vascular endothelial cells, unlike vascular endothelial progenitor cells in bone marrow, were shown to have the potential of contributing as vascular endothelial cells for a long time. Moreover, the present inventors found that such cells, highly capable of generating vascular endothelial cells, are present as vascular endothelial cells with a high drug efflux capacity in preexisting blood vessels.

Furthermore, the present inventors precisely analyzed these vascular endothelial cells, which are highly capable of generating vascular endothelial cells, to clarify their stem cell-like characteristics. For this purpose, the present inventors isolated surface markers expressed on these vascular endothelial cells and found that CD157 and CD200 are expressed on the vascular endothelial cells (Non-Patent Literature 2 and Patent Literature 1). Also found was that a population of CD200-positive and CD157-positive vascular endothelial cells has the abilities to divide themselves (self-renew) in an undifferentiated state as well as to ultimately generate a large number of CD200-negative and CD157-negative differentiated vascular endothelial cells. In addition, blood vessels regenerated from these vascular endothelial cells were shown to be maintained without regression for a long time. Specifically, the regenerated blood vessels retained functions such as blood supply as well as long-term production of molecules that are supposed to be secreted by vascular endothelial cells (e.g., coagulation factor VIII etc.). The vascular endothelial cells possessing stem cell properties found by the present inventors are hereinafter referred to as "vascular endothelial stem cells".

Induction of vascular regeneration requires the use of vascular endothelial cells possessing stem cell properties as described above. Transplantation of such vascular endothelial stem cells can potentially provide therapeutic benefits to patients without vascular reserve. However, there are a very small number of vascular endothelial stem cells in the body, and it is very inefficient to harvest the patient's own vascular endothelial stem cells from each patient who is to undergo vascular regeneration. Additionally, the patient's own vascular endothelial stem cell function may be impaired. Therefore, there is a need to develop a method for artificially producing vascular endothelial stem cells from some type of cells.

### CITATION LIST

### Patent Literature

Patent Literature 1: WO 2019/098264 A1

### Non-Patent Literature

Non-Patent Literature 1:
   Naito H. et al., Identification and characterization of a resident vascular stem/progenitor cell population in preexisting blood vessels, EMBO J. 2012 Feb 15; 31 (4): 842-55.
Non-Patent Literature 2:
   Wakabayashi T. et al., CD157 Marks Tissue-Resident Endothelial Stem Cells with Homeostatic and Regenerative Properties, Cell Stem Cell. 2018 Mar 1; 22 (3): 384-397.

### SUMMARY OF INVENTION

### TECHNICAL PROBLEM

An object of the present invention is to provide a method for artificially producing a vascular endothelial stem cell from a non-vascular endothelial stem cell.

### SOLUTION TO PROBLEM

The present invention includes the following to achieve the above-mentioned objects.
[1] A method for producing a vascular endothelial stem cell, comprising the step of bringing a vascular endothelial cell possessing no stem cell properties into contact with a factor secreted by an organ of a neonatal or juvenile mammal.
[2] The method according to the above [1], wherein the step is performed by either of the following:
   (1) transplanting the vascular endothelial cell possessing no stem cell properties into the organ of the neonatal or juvenile mammal, wherein the mammal is a non-human mammal, or
   (2) culturing the vascular endothelial cell possessing no stem cell properties in a culture system containing the factor secreted by the organ of the neonatal or juvenile mammal.
[3] The method according to the above [1] or [2], wherein the neonatal or juvenile mammal is a neonatal mammal.
[4] The method according to any one of the above [1] to [3], wherein the vascular endothelial cell possessing no stem cell properties is a cell obtained from a neonatal or juvenile mammal.
[5] The method according to the above [4], wherein the vascular endothelial cell possessing no stem cell properties is a cell obtained from a neonatal mammal.
[6] The method according to any one of the above [1] to [5], wherein the organ is liver.
[7] An agent for inducing a vascular endothelial stem cell, comprising a factor secreted by an organ of a neonatal or juvenile mammal.
[8] The agent for inducing a vascular endothelial stem cell according to the above [7], wherein the agent comprises a culture supernatant of cells prepared from the organ of the neonatal or juvenile mammal or an extract of the organ of the neonatal or juvenile mammal, and wherein the culture supernatant and the extract contain the factor.
[9] The agent for inducing a vascular endothelial stem cell according to the above [7] or [8], wherein the neonatal or juvenile mammal is a neonatal mammal.
[10] The agent for inducing a vascular endothelial stem cell according to any one of the above [7] to [9], wherein the organ is liver.

### ADVANTAGEOUS EFFECTS OF INVENTION

The present invention provides a method for artificially producing a vascular endothelial stem cell from a vascular endothelial cell possessing no stem cell properties.

### BRIEF DESCRIPTION OF DRAWINGS

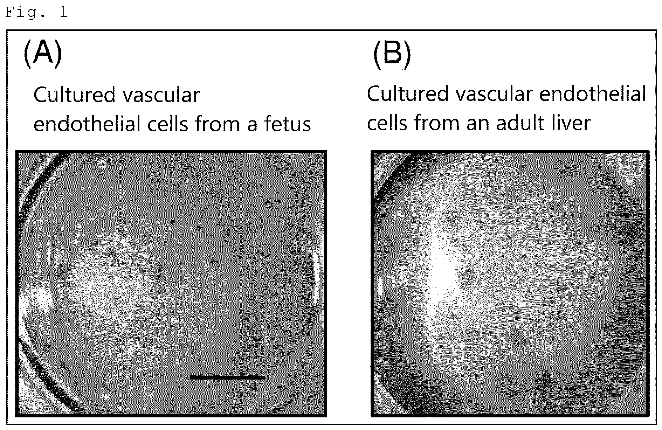

Fig. 1 shows vascular endothelial cells (CD31-positive and CD45-negative cells) that were obtained by flow cytometry from a mouse body and cultured in a culture system using OP9 stromal cells as feeder cells. Fig. 1A shows vascular endothelial cells cultured after obtained from a fetal mouse at embryonic day 15, and Fig. 1B shows vascular endothelial cells cultured after obtained from an 8-week-old adult mouse liver.

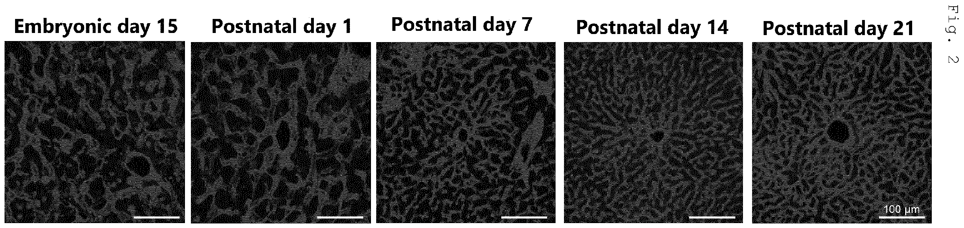

Fig. 2 shows the results of immunostaining with anti-CD31 antibody on cryosections of livers excised from a fetal mouse at embryonic day 15 and neonatal to juvenile mice at postnatal days 1, 7, 14, and 21.

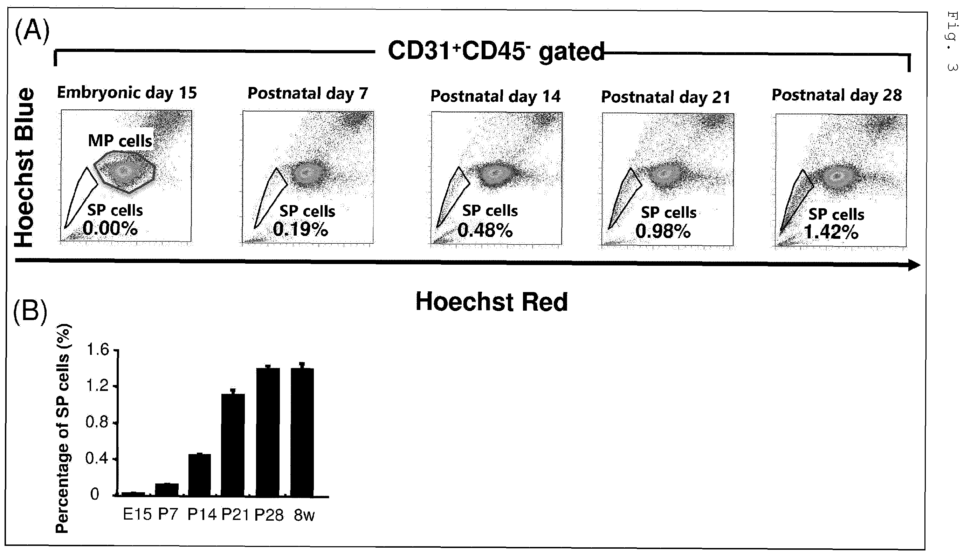

Fig. 3 shows the results of Hoechst staining and flow cytometric analysis of vascular endothelial cells (CD31-positive and CD45-negative cells) obtained by flow cytometry from livers of fetal mice at embryonic day 15 and neonatal to juvenile mice at postnatal days 7, 14, 21, and 28. Fig. 3A shows the results of Hoechst staining and flow cytometric analysis of the cells, and Fig. 3B is a graph showing the percentage of a fraction with high drug efflux capacity (SP cell fraction).

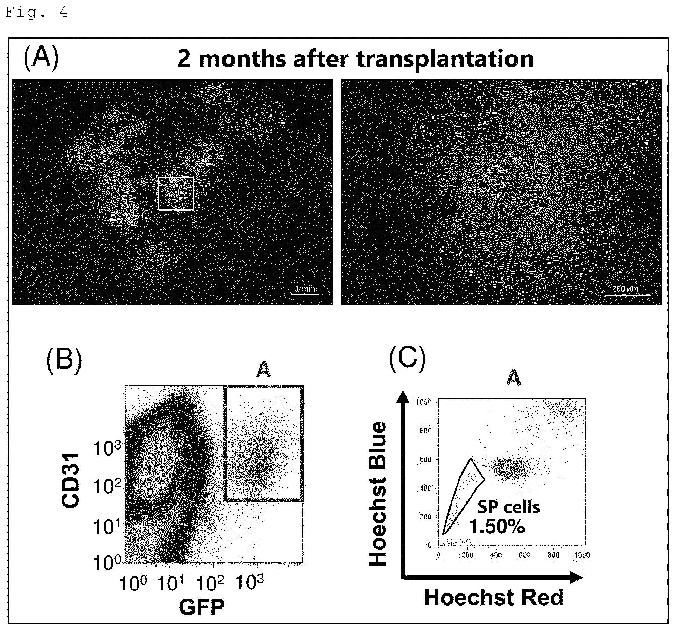

Fig. 4 shows the results of analysis of the liver from a liver injury model mouse (C57BL/6) which underwent intrahepatic transplantation at postnatal day 7 with vascular endothelial cells (CD31-positive and CD45-negative cells) in a fraction with low drug efflux capacity (MP cell fraction) obtained from the liver of a postnatal day 1 green mouse (C57BL/6-Tg(CAG-EGFP)) and was fed for two months. Fig. 4A shows the liver observed under a fluorescence stereomicroscope after laparotomy, Fig. 4B shows the results of flow cytometric analysis for the presence of CD31-positive and GFP-positive cells in the liver of Fig. 4A, and Fig. 4C shows the results of flow cytometric analysis for the presence of SP cells in the CD31-positive and GFP-positive cells of Fig. 4B.

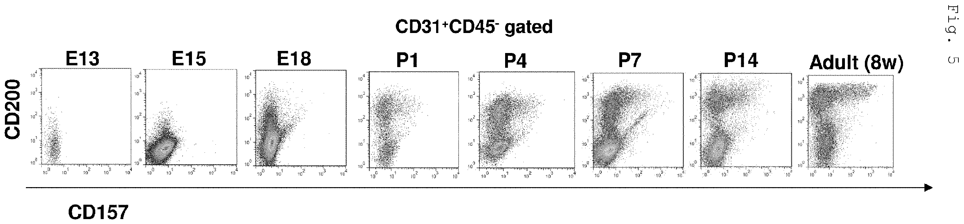

Fig. 5 shows the results of flow cytometric analysis for the presence of CD200-positive and CD157-positive cells in vascular endothelial cells (CD31-positive and CD45-negative cells) obtained by flow cytometry from livers of fetal mice at embryonic days 13, 15, and 18, neonatal mice at postnatal days 1, 4, 7, and 14, and adult mice at week 8.

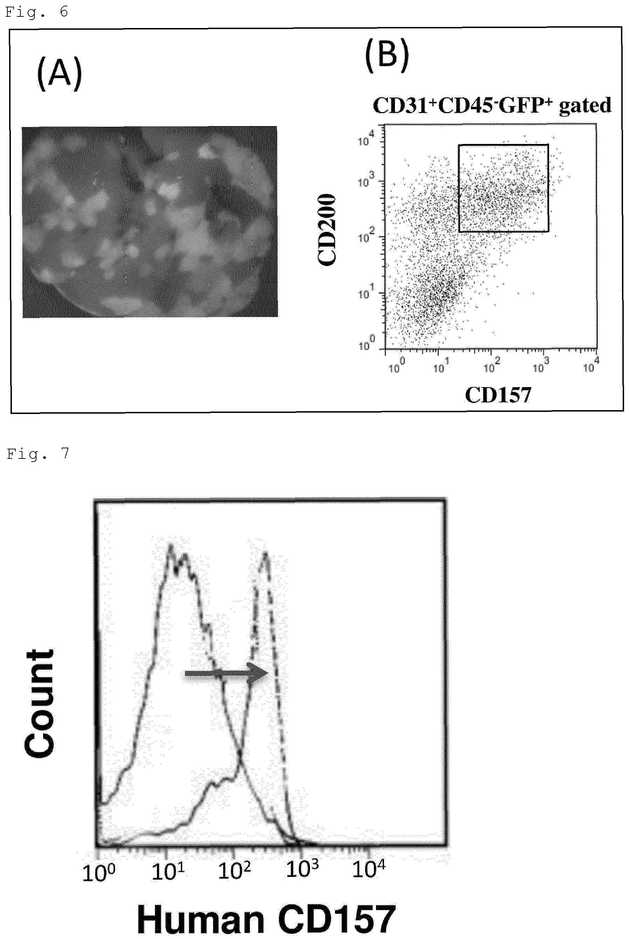

Fig. 6 shows the results of analysis of the liver from a mouse which underwent intrahepatic transplantation at postnatal day 7 with CD200-negative and CD157-negative vascular endothelial cells (CD31-positive and CD45-negative cells) obtained from the liver of an embryonic day 15 green mouse (C57BL/6-Tg (CAG-EGFP)) and was fed for two months. Fig. 6A shows the liver observed under a fluorescence stereomicroscope after laparotomy, and Fig. 6B shows the results of flow cytometric analysis for the presence of CD200-positive and CD157-positive cells in the CD31-positive, CD45-negative, and GFP-positive cells in the liver of Fig. 6A.
Fig. 7 shows the results of flow cytometric analysis of CD157 expression in human umbilical vein vascular endothelial cells (HUVECs) before and after co-culture with cells prepared from the livers of neonatal mice at postnatal day 4.

### DESCRIPTION OF EMBODIMENTS

### Method for a producing vascular endothelial stem cell

The present invention provides a method for producing a vascular endothelial stem cell. The method for producing a vascular endothelial stem cell of the present invention (hereinafter referred to as the "production method of the present invention") comprises the step of bringing a vascular endothelial cell possessing no stem cell properties into contact with a factor secreted by an organ of a neonatal or juvenile mammal. The organ of the neonatal or juvenile mammal may be a neonatal mammalian organ.

As used herein, the vascular endothelial stem cell refers to a cell that has the abilities to divide itself in an undifferentiated state (self-renewal ability) and to differentiate into a vascular endothelial cell. The generation of the vascular endothelial stem cell by the production method of the present invention can be confirmed by testing for the presence of a vascular endothelial cell that is capable of forming a single-cell-derived colony. That is, a vascular endothelial cell having a high colony-forming ability can be identified as the vascular endothelial stem cell. In addition, the cell generated by the production method of the present invention may be further tested to confirm that it has a high drug efflux capacity and/or is a CD200-positive and CD157-positive cell. Testing for drug efflux capacity can be performed using Hoechst staining, for example. Whether the cell is a CD200-positive and CD157-positive cell can be determined, for example, by staining the cell with anti-CD200 and anti-CD157 antibodies.

Vascular endothelial cells are flattened cells forming a monolayer lining the lumen of blood vessels and can be defined as CD31-positive and CD45-negative cells. A vascular endothelial cell obtained as a CD31-positive and CD45-negative cell by the usual method is suitable for use as the vascular endothelial cell possessing no stem cell properties in the production method of the present invention.

The vascular endothelial cell possessing no stem cell properties is simply required to a vascular endothelial cell from a mammal. The mammal is not particularly limited, and examples include humans, monkeys, cattle, pigs, sheep, goats, dogs, cats, mice, rats, and rabbits. When the mammal is a human, the vascular endothelial stem cell produced by the production method of the present invention can be safely used for cell therapy and other applications in humans.

The vascular endothelial cell possessing no stem cell properties can be prepared from any organ. The organ may be, for example, liver, retina, brain, heart, skin, muscle (skeletal muscle), lung, kidney, placenta, umbilical cord, adipose tissue, etc. Liver and umbilical cord are preferable. The method for preparing the vascular endothelial cell possessing no stem cell properties is not particularly limited. For example, an isolated organ is digested and dissociated with a commercially available cell-dissociation reagent to prepare a cell suspension, and this cell suspension is stained with anti-CD31 and anti-CD45 antibodies and then subjected to flow cytometry to collect CD31-positive and CD45-negative cells.

The vascular endothelial cell possessing no stem cell properties is simply required to be a vascular endothelial cell obtained from a mammal, and when to obtain the cell from the mammal is not particularly limited. The vascular endothelial cell possessing no stem cell properties may be a vascular endothelial cell obtained from a neonatal or juvenile mammal and is preferably a vascular endothelial cell obtained from a neonatal mammal. As used herein, the neonatal period refers to a period from birth to weaning, and the juvenile period refers to a period from the end of weaning to the acquisition of reproductive capacity. The present inventors have demonstrated that vascular endothelial cells from neonatal mice are more likely to be reprogrammed into vascular endothelial stem cells than vascular endothelial cells from adult mice.

In the step of bringing a vascular endothelial cell possessing no stem cell properties into contact with a factor secreted by an organ of a neonatal or juvenile mammal, for example, the vascular endothelial cell may be transplanted into the organ of the neonatal or juvenile mammal, wherein the mammal is a non-human mammal. The non-human mammal is not particularly limited, and examples include monkeys, cattle, pigs, sheep, goats, dogs, cats, mice, rats, and rabbits. Pigs and sheep are preferable. The organ is not particularly limited and may be, for example, liver, retina, brain, heart, skin, muscle (skeletal muscle), lung, kidney, placenta, adipose tissue, etc. Liver is preferable. For transplantation, for example, a host animal is laparotomized under anesthesia, and the vascular endothelial cell possessing no stem cell properties is introduced into the target organ by injection or other means.

In the step of bringing a vascular endothelial cell possessing no stem cell properties into contact with a factor secreted by an organ of a neonatal or juvenile mammal, for example, the vascular endothelial cell may be cultured in a culture system containing the factor secreted by the organ of the neonatal or juvenile mammal. Specifically, the vascular endothelial cell possessing no stem cell properties may be co-cultured with cells prepared from the organ of the neonatal or juvenile mammal. For cell preparation from the organ, an isolated organ may be digested and dissociated with a commercially available cell-dissociation reagent, for example. For co-culture, the vascular endothelial cell possessing no stem cell properties and the cells prepared from the organ are cultured in the same culture vessel, for example. In this case, to prevent direct contact between the vascular endothelial cell possessing no stem cell properties and the cells prepared from the organ, a culture vessel with culture inserts or other devices placed therein may be used for co-culture.

In a possible alternative procedure, cells prepared from the organ of the neonatal or juvenile mammal are cultured in a suitable culture medium, and the culture supernatant of the cells is collected and added to a culture medium for the vascular endothelial cell possessing no stem cell properties. Still alternatively, an extract of the organ of the neonatal or juvenile mammal may be prepared and added to a culture medium for the vascular endothelial cell possessing no stem cell properties. For example, an organ homogenate supernatant is suitable for use as the extract of the organ of the neonatal or juvenile mammal.

The duration for which the vascular endothelial cell possessing no stem cell properties is in contact with the factor secreted by the organ of the neonatal or juvenile mammal is not particularly limited. An appropriate duration of time can be determined in preliminary studies etc. so that the desired vascular endothelial stem cell can be obtained. For example, in the case of transplantation into the organ of the neonatal or juvenile non-human mammal, the contact duration may be 1 day or more, 2 days or more, 3 days or more, or 4 days or more, and may be 100 days or less, 50 days or less, 10 days or less, or 5 days or less. For example, in the case of co-culture with the cells prepared from the organ of the neonatal or juvenile non-human mammal, the contact duration may be 1 day or more, 2 days or more, 3 days or more, or 4 days or more, and may be 100 days or less, 50 days or less, 10 days or less, or 5 days or less.

The contact of the vascular endothelial cell possessing no stem cell properties with the factor secreted by the organ of the neonatal or juvenile mammal for a certain duration of time results in the reprogramming of the vascular endothelial cell possessing no stem cell properties into a vascular endothelial stem cell. When transplantation into an organ of a non-human mammal has been employed, the generation of the vascular endothelial stem cell can be confirmed as follows: the transplantation site is resected and then digested and dissociated with a commercially available cell-dissociation reagent to prepare a cell suspension, and this cell suspension is tested for the presence of a vascular endothelial cell having a high colony-forming ability (CD31-positive and CD45-negative cell). When a culture system containing a factor secreted by an organ of a neonatal or juvenile mammal has been employed, the generation of the vascular endothelial stem cell can be confirmed by testing for the presence of a vascular endothelial cell having a high colony-forming ability (CD31-positive and CD45-negative cell) in the cells after the culture period. In addition, the vascular endothelial cell having a high colony-forming ability may be further tested to confirm that it has a high drug efflux capacity and/or is a CD200-positive and CD157-positive cell. The generated vascular endothelial stem cell can be collected, for example, by isolating and proliferating the cell that has formed a colony. Alternatively, the generated vascular endothelial stem cell can be collected based on the following indicators: high drug efflux capacity and/or positivity for both CD200 and CD157.

### Agent for inducing a vascular endothelial stem cell

The present invention provides an agent for inducing a vascular endothelial stem cell which agent comprises a factor secreted by an organ of a neonatal or juvenile mammal. The organ of the neonatal or juvenile mammal may be a neonatal mammalian organ.

The mammal is not particularly limited, and examples include humans, monkeys, cattle, pigs, sheep, goats, dogs, cats, mice, rats, and rabbits. The mammal may be a human. The organ is not particularly limited and may be, for example, liver, retina, brain, heart, skin, muscle (skeletal muscle), lung, kidney, placenta, fat, etc. The organ may be liver.

The agent for inducing a vascular endothelial stem cell may be in any form that comprises the factor secreted by the organ of the neonatal or juvenile mammal. For example, the agent for inducing a vascular endothelial stem cell may comprise a culture supernatant of cells prepared from the organ of the neonatal or juvenile mammal, or comprise an extract of the organ of the neonatal or juvenile mammal. The culture supernatant and the extract contain the factor secreted by the organ. The culture supernatant can be obtained, for example, by digesting and dissociating an isolated organ with a commercially available cell-dissociation reagent and then culturing the dissociated cells in a suitable culture medium. The organ extract can be obtained, for example, by homogenizing the organ and then centrifuging the homogenate.

The agent for inducing a vascular endothelial stem cell can be added to a culture medium for culturing vascular endothelial cells possessing no stem cell properties. The amount of the agent added is recommended to be set as appropriate for the culture conditions etc.

The present invention further includes the following.

A method for inducing a vascular endothelial stem cell, comprising the step of culturing a vascular endothelial cell possessing no stem cell properties in a culture medium containing a factor secreted by an organ of a neonatal or juvenile mammal.

Use of a factor secreted by an organ of a neonatal or juvenile mammal for induction of a vascular endothelial stem cell.

Use of a culture supernatant of cells prepared from an organ of a neonatal or juvenile mammal for induction of a vascular endothelial stem cell.

Use of an extract of an organ of a neonatal or juvenile mammal for induction of a vascular endothelial stem cell.

Use of a factor secreted by an organ of a neonatal or juvenile mammal for production of an agent for inducing a vascular endothelial stem cell.

Use of a culture supernatant of cells prepared from an organ of a neonatal or juvenile mammal for production of an agent for inducing a vascular endothelial stem cell.

Use of an extract of an organ of a neonatal or juvenile mammal for production of an agent for inducing a vascular endothelial stem cell.

### EXAMPLES

Hereinafter, the present invention will be described in detail by examples, but the present invention is not limited thereto.

### Example 1: Examination of stem cell properties in vascular endothelial cells during fetal and postnatal periods 1-1 Experimental methods

Fetal C57BL/6 mice at embryonic day 15 (E15) and adult C57BL/6 mice at postnatal week 8 (8W) (Japan SLC) were used. The whole body of each E15 fetus and the liver excised from each 8W mouse were minced as finely as possible using ophthalmic scissors. The minced tissue was immersed in a mixture of Dispase II (Roche Applied Science), collagenase (Wako), and type II collagenase (Worthington Biochemical Corporation) with shaking at 37°C to digest the extracellular matrix. The digest mixture containing cells was passed through a filter with a pore size of 40 µm to yield a suspension of dissociated cells. Erythrocytes were lysed with ACK (Ammonium-Chloride-Potassium) buffer (0.15MNH₄Cl, 10 mM KHCO₃, and 0.1 mM Na₂-EDTA), and the remaining cells were used for the following experiments.

The prepared cells were immunofluorescently stained and analyzed by flow cytometry. The monoclonal antibodies used were an anti-CD31 antibody (clone MEC13.3, BD Biosciences) and an anti-CD45 antibody (clone 30-F11, BD Biosciences). After staining, propidium iodide (PI, 2 µg/mL, Sigma-Aldrich) was added to the cells to stain the nuclei of dead cells, and dead cells were removed. For flow cytometric analysis, SOAP FACSAria (BD Biosciences) and FlowJo Software (Treestar Software) were used. CD31-positive and CD45-negative vascular endothelial cells obtained by flow cytometry were cultured in a culture system using OP9 stromal cells (RIKEN cell bank) as feeder cells. More specifically, 5,000 cells of each type of cells were seeded onto culture plates with a diameter of 3 cm and cultured for 7 days.

### 1-2 Results

The results are shown in Fig. 1. Fig. 1A shows vascular endothelial cells cultured after obtained from a fetal mouse, and Fig. 1B shows vascular endothelial cells cultured after obtained from an adult mouse liver. Among vascular endothelial cells obtained from the fetuses, a small number of cells were capable of proliferating and forming colonies. On the other hand, among the vascular endothelial cells collected from the adult livers, some cells were capable of forming very large colonies. These results indicate that vascular endothelial stem cells do not exist during the fetal period but may be generated after birth.

### Example 2: Examination of sinusoidal blood vessel formation in liver

### 2-1 Experimental method

Livers were excised from fetal C57BL/6 mice at embryonic day 15 and neonatal to juvenile C57BL/6 mice at postnatal days 1, 7, 14, and 21 (SLC Japan) . Cryosections were prepared from the livers according to the usual method. The cryosections were immunostained with an anti-CD31 antibody (clone MEC13.3, BD Biosciences). A PE-labeled anti-rat IgG (BD Biosciences) was used as a secondary antibody.

### 2-2 Results

The results are shown in Fig. 2. In the livers of juvenile mice at postnatal day 21, sinusoidal blood vessels were formed extending radially from the central vein. These radially formed liver-specific vascular structures were not formed in the livers of mice during the fatal period, but were shown to be gradually formed after birth. Since the present inventors previously revealed that sinusoidal blood vessels, which are unique vascular structures in the liver, are induced from vascular endothelial stem cells (Wakabayashi T. et al., Cell Stem Cell. 2018 Mar 1; 22 (3): 384-397), the results herein indicate that vascular endothelial stem cells do not exist during the fetal period but may be generated after birth.

### Example 3: Analysis of emerging pattern of vascular endothelial stem cell-containing fraction (SP cell fraction), which can be isolated using high drug efflux capacity

### 3-1 Experimental method

Livers were excised from fetal C57BL/6 mice at embryonic day 15 (E15), neonatal to juvenile C57BL/6 mice at postnatal days 1 (P1), 7 (P7), 14 (P14), 21 (P21), and 28 (P28), and adult C57BL/6 mice at week 8 (8W) (SLC Japan) . As in Example 1, each excised liver was minced as finely as possible using ophthalmic scissors. The minced tissue was immersed in a mixture of Dispase II (Roche Applied Science), collagenase (Wako), and type II collagenase (Worthington Biochemical Corporation) with shaking at 37°C to digest the extracellular matrix. The digest mixture containing cells was passed through a filter with a pore size of 40 µm to yield a suspension of dissociated cells. The cell suspension was subjected to Hoechst staining. For Hoechst staining, 1 × 10⁶ cells were suspended in 1 ml of Hoechst-containing medium (DMEM (Sigma-Aldrich) containing 2% FBS (Sigma-Aldrich), 1 mM HEPES (Gibco), and 5 µg/mL Hoechst 33342 (Sigma-Aldrich)) and incubated at 37°C for 90 min. For immunofluorescence staining, the same anti-CD31 and anti-CD45 antibodies as those in Example 1 were used. After staining, propidium iodide (PI, 2 µg/mL, Sigma-Aldrich) was added to the cells to stain the nuclei of dead cells, and dead cells were removed. CD31-positive, CD45-negative, and PI-negative cells (vascular endothelial cells without dead cells) were subjected to Hoechst staining analysis by flow cytometry. For flow cytometric analysis, SOAP FACSAria (BD Biosciences) and FlowJo Software (Treestar Software) were used.

### 3-2 Results

The results are shown in Fig. 3. Fig. 3A shows the representative results of the flow cytometric analysis of the cells during fetal and neonatal to juvenile periods, and Fig. 3B is a graph showing the percentage of the SP cell fraction. In fetal vascular endothelial cells, cells in the SP cell fraction (SP cells), including vascular endothelial stem cells, were not observed, and only non-vascular endothelial stem cells defined as the MP cell fraction (MP cells) were observed. The SP cells gradually emerged after birth, and in 21-day-old (P21) juvenile mice, the percentage of the SP cells was about 1%, which was comparable to the level in 8-week-old (8W) adult mice. These results show that vascular endothelial stem cells are generated in the intravital environment after birth.

### Example 4: MP cells change into SP cells in liver environment 4-1 Experimental method

C57BL/6 mice and C57BL/6-Tg(CAG-EGFP) mice (hereinafter referred to as "green mice") were purchased from SLC Japan and used in the following experiments. Livers were excised from green mice at postnatal day 1 (1 day of age) and week 8 (8 weeks of age), and cell suspensions were prepared as described in Example 3. Hoechst staining and immunofluorescence staining (anti-CD31 antibody, anti-CD45 antibody, PI) were performed as described in Example 3, and MP cells were collected from vascular endothelial cells by flow cytometry. The collected MP cells (1 × 10⁵ cells) were transplanted into liver injury model mice. For preparation of the liver vascular injury model mice, C57BL/6 mice at postnatal day 7 (7 days of age) and week 8 (8 weeks of age) were intraperitoneally treated with monocrotaline (Sigma-Aldrich) at a dose of 300 mg/kg and systemically irradiated with 30 rads/g on the same day. Two months after the MP cell transplantation, the mice were laparotomized under anesthesia, and the livers were observed under a fluorescence stereomicroscope (Leica). Furthermore, the livers were excised, and cell suspensions were prepared as described in Example 3. Hoechst staining and immunofluorescence staining (anti-CD31 antibody, anti-CD45 antibody, PI) were performed, and the presence of SP cells was assessed by flow cytometry.

### 4-2 Results

Fig. 4 shows the results of transplantation of MP cells from a 1-day-old green mouse liver into a 7-day-old liver injury model mouse. Fig. 4A shows the liver of the liver injury model mouse observed two months after MP cell transplantation, Fig. 4B shows the results of the flow cytometric analysis for the presence of CD31-positive and GFP-positive cells in the mouse liver of Fig. 4A, and Fig. 4C shows the results of the flow cytometric analysis for the presence of SP cells in the CD31-positive and GFP-positive cells of Fig. 4B. As shown in Fig. 4A, GFP-positive cells transplanted from the green mouse were observed in the liver of the liver injury model mouse. As shown in Fig. 4B, a large number of CD31-positive and GFP-positive cells were detected, indicating that the vascular endothelial cells from the green mouse formed blood vessels in the host liver. Furthermore, as shown in Fig. 4C, SP cells were present at 1.5% in the CD31-positive and GFP-positive cells. These results demonstrate that MP cells are capable of being reprogrammed into SP cells under an appropriate environment.

Table 1 shows a summary of the results of transplantation of GFP-positive MP cells from neonatal (1-day-old) and adult (8-week-old) mice into neonatal (7-day-old) and adult (8-week-old) liver injury model mice, respectively. The MP cells transplanted into the livers of adult mice were not reprogrammed into SP cells, but the MP cells transplanted into the livers of neonatal mice were reprogrammed into SP cells. In addition, MP cells from neonatal mice were more likely to be reprogrammed into SP cells than MP cells from adult mice. These results indicate that neonatal livers abundantly contain factors capable of reprogramming MP cells into SP cells, while adult livers hardly contain such factors. Furthermore, MP cells in neonatal livers are seemingly more susceptible to reprogramming factors than MP cells in adult livers and more likely to be reprogrammed into SP cells.

**[Table 1]**

| Source of vascular endothelial cells | Host tissue | Frequency of reprogramming of vascular endothelial cells into vascular endothelial stem cells |
|---|---|---|
| Adult liver | Adult liver | Almost never |
| Adult liver | Neonatal liver | Very infrequent |
| Neonatal liver | Adult liver | Almost never |
| Neonatal liver | Neonatal liver | Very frequent |

### Example 5: Analysis of CD157-positive and CD200-positive vascular endothelial stem cell generation process 5-1 Experimental method

Livers were excised from fetal C57BL/6 mice at embryonic days 13 (E13), 15 (E15), and 18 (E18), neonatal C57BL/6 mice at postnatal days 1 (P1), 4 (P4), 7 (P7), and 14 (P14), and adult C57BL/6 mice at week 8 (8W) (SLC Japan), and cell suspensions were prepared as described in Examples 1 and 3. The monoclonal antibodies used here were an anti-CD31 antibody (clone MEC13.3, BD Biosciences), an anti-CD45 antibody (clone 30-F11, BD Biosciences), an anti-CD157 antibody (clone BP3, BioLegend), and an anti-CD200 antibody (clone OX90, BioLegend). After staining, propidium iodide (PI, 2 µg/mL, Sigma-Aldrich) was added to the cells to stain the nuclei of dead cells, and dead cells were removed. For flow cytometric analysis, SOAP FACSAria (BD Biosciences) and FlowJo Software (Treestar Software) were used.

### 5-2 Results

The results are shown in Fig. 5. CD31-positive and CD45-negative cells in the liver were gated as vascular endothelial cells in the liver, and the expression of CD200 and CD157 was analyzed by flow cytometry. The result shows that all vascular endothelial cells were CD200-negative and CD157-negative cells until E15, while a small number of CD200-positive and CD157-negative cells emerged at E18. After birth, a CD200-positive and CD157-negative cell population began to separate clearly from a CD200-negative and CD157-negative cell population at P1. On the other hand, a CD200-positive and CD157-positive cell population was almost absent. At P4, a small number of CD200-positive and CD157-positive cells emerged, and at P7 and P14, the number of CD200-positive and CD157-positive cells was gradually increased. In 8W adult mice, 4 to 6% of the entire vascular endothelial cell population was CD200-positive and CD157-positive cells. Subsequently, CD200-positive and CD157-positive vascular endothelial stem cells were continuously present in the liver at an approximately constant proportion, but the data are not shown here. These results were consistent with those obtained by the Hoechst efflux assay in Example 3 for vascular endothelial stem cells in SP cells. In summary, there are no vascular endothelial stem cells in the liver during the fetal period, but just before birth, some vascular endothelial progenitor cells emerge, which cells partially possess stem cell properties but are more differentiated than stem cells, leading to generation of vascular endothelial stem cells in the liver after birth.

### Example 6: CD157-negative and CD200-negative vascular endothelial cells change to vascular endothelial stem cells in neonatal liver environment

### 6-1 Experimental method

Livers were excised from green mice at embryonic day 15 (E15), and cell suspensions were prepared as described in Example 3. The cell suspensions were stained with the same anti-CD31, anti-CD45, anti-CD157, and anti-CD200 antibodies as those used in Example 5, and flow cytometric analysis was performed to collect CD200-negative and CD157-negative vascular endothelial cells (CD31-positive and CD45-negative cells) . The collected cells were transplanted into the livers of liver injury model mice (C57BL/6) at postnatal day 7 (7 days of age) as described in Example 4. The liver injury model mice were prepared as described in Example 4. Two months after transplantation, the mice were laparotomized under anesthesia, and the livers were observed under a fluorescence stereomicroscope (Leica). Furthermore, the livers were excised, and cell suspensions were prepared as described in Example 3. The cell suspensions were stained with the same anti-CD31, anti-CD45, and anti-CD157 antibodies as those used in Example 5, and flow cytometric analysis was performed.

### 6-2 Results

The results are shown in Fig. 6. Fig. 6A shows a host mouse liver observed two months after transplantation, and Fig. 6B shows the results of the flow cytometric analysis for the presence of CD200-positive and CD157-positive cells in CD31-positive, CD45-negative, and GFP-positive cells in the host mouse liver of Fig. 6A. As shown in Fig. 6A, a large number of GFP-positive cells from a green mouse were observed in the host mouse liver, indicating that CD200-negative and CD157-negative vascular endothelial cells (CD31-positive, CD45-negative, and GFP-positive cells) from the green mouse fetus are able to acquire the potential for contribution to sinusoidal blood vessels in the neonatal liver environment. As shown in Fig. 6B, some of the transplanted CD200-negative and CD157-negative vascular endothelial cells from the green mouse fetus were reprogrammed into CD200-positive and CD157-positive vascular endothelial stem cells.

### Example 7: Change from human vascular endothelial cells to vascular endothelial stem cells

### 7-1 Experimental method

Human umbilical vein endothelial cells (HUVECs) (Lonza) were used as human vascular endothelial cells just after birth. HUVECs were cultured in HuMedia-EG2 (KURABO, Osaka, Japan) culture medium. HUVECs were seeded onto 6-well plates at 1.0 × 10⁵ cells/well, and culture inserts (Corning, NY, USA) with a pore size of 1.0 µm were set into the wells. Livers were excised from neonatal mice (C57BL/6) at postnatal day 4, and cell suspensions were prepared as described in Example 1. The cell suspensions were seeded at 2.0 × 10⁶ cells per culture insert and co-cultured with the HUVECs for 5 days without direct contact between the HUVECs and the neonatal mouse cells. After co-culture, the HUVECs were collected, stained with a CD157 antibody, and analyzed by flow cytometry.

### 7-2 Results

The results are shown in Fig. 7. HUVECs before co-culture were CD157 negative, but were induced to express CD157 by co-culture with the cells from the neonatal mouse liver and detected as CD157 positive. These results demonstrate that cells in the neonatal liver have the ability to induce CD157 and differentiate endothelial cells into endothelial stem cells and that humoral factors secreted by the liver may be involved in this differentiation process.

The present invention is not limited to the particular embodiments and examples described above, and various modifications can be made within the scope of the appended claims. Other embodiments provided by suitably combining technical means disclosed in separate embodiments of the present invention are also within the technical scope of the present invention. All the academic publications and patent literature cited in the description are incorporated herein by reference.

## Claims

1. A method for producing a vascular endothelial stem cell, comprising the step of bringing a vascular endothelial cell possessing no stem cell properties into contact with a factor secreted by an organ of a neonatal or juvenile mammal.

2. The method according to claim 1, wherein the step is performed by either of the following:
(1) transplanting the vascular endothelial cell possessing no stem cell properties into the organ of the neonatal or juvenile mammal, wherein the mammal is a non-human mammal, or
(2) culturing the vascular endothelial cell possessing no stem cell properties in a culture system containing the factor secreted by the organ of the neonatal or juvenile mammal.

3. The method according to claim 1 or 2, wherein the neonatal or juvenile mammal is a neonatal mammal.

4. The method according to any one of claims 1 to 3, wherein the vascular endothelial cell possessing no stem cell properties is a cell obtained from a neonatal or juvenile mammal.

5. The method according to claim 4, wherein the vascular endothelial cell possessing no stem cell properties is a cell obtained from a neonatal mammal.

6. The method according to any one of claims 1 to 5, wherein the organ is liver.

7. An agent for inducing a vascular endothelial stem cell, comprising a factor secreted by an organ of a neonatal or juvenile mammal.

8. The agent for inducing a vascular endothelial stem cell according to claim 7, wherein the agent comprises a culture supernatant of cells prepared from the organ of the neonatal or juvenile mammal or an extract of the organ of the neonatal or juvenile mammal, and wherein the culture supernatant and the extract contain the factor.

9. The agent for inducing a vascular endothelial stem cell according to claim 7 or 8, wherein the neonatal or juvenile mammal is a neonatal mammal.

10. The agent for inducing a vascular endothelial stem cell according to any one of claims 7 to 9, wherein the organ is liver.
